# EUROPEAN PATENT SPECIFICATION

(11) **EP 4 058 102 B1**
(45) Date of publication and mention of the grant of the patent: **01.10.2025**
(21) Application number: 20789606.9
(22) Date of filing: 13.10.2020
(51) Int. Cl.: A61M 5/20

(54) **MEDICAMENT DELIVERY DEVICE**
MEDIKAMENTENVERABREICHUNGSVORRICHTUNG
DISPOSITIFS D'ADMINISTRATION DE MÉDICAMENTS

(30) Priority: 11.11.2019 US 201962933532 P; 11.12.2019 EP 19215378
(43) Date of publication of application: 21.09.2022
(73) Proprietor: SHL Medical AG, 6302 Zug (CH)
(72) Inventor: CHEN, Yuehjen, Taoyuan City, 338 (TW); FROST, Jason, Boca Raton, Florida 33431 (US)
(86) International application number: PCT/EP2020/078696
(87) International publication number: WO 2021/094047

(56) References cited:
- WO-A1-2013/178512
- WO-A1-2017/198383
- WO-A2-2013/034984
- GB-A- 2 560 558
- US-A1- 2008 147 006
- US-A1- 2015 273 160
- US-B2- 10 525 201

## Description

### TECHNICAL FIELD

The present disclosure generally relates to medicament delivery devices.

### BACKGROUND

Administration of some medicaments require an injection at a certain depth. Therefore, medicament delivery devices may be provided with an auto-penetration functionality. Hereto, a sub-assembly comprising the delivery member, typically a needle, is moved forward a predetermined distance automatically when the medicament delivery device is triggered. This forward movement causes the delivery member to be inserted to the required depth into the site of delivery.

WO2017/198383 A1 discloses a medicament delivery device with auto-penetration functionality. This document discloses an administration mechanism for a medicament delivery device, comprising: a linearly displaceable medicament delivery member cover, an actuator sleeve, and an actuator configured to be received by the actuator sleeve. The medicament delivery member cover is configured to axially displace the actuator sleeve from an initial position to a distally displaced position thereby displacing the actuator sleeve relative to the actuator. The administration mechanism further comprises a rotator having a guide structure and configured to be received by the actuator, a plunger holder configured to be received by and engage with the rotator, and a plunger rod. The rotator and plunger holder are configured to be proximally biased, and the plunger is rod configured to be received by and engage with the plunger holder in the initial position of the actuator sleeve. The plunger rod is configured to be proximally biased, wherein the actuator is configured to engage with the plunger holder in the initial position of the actuator sleeve, thereby preventing the plunger holder from proximal displacement relative to the actuator, wherein the actuator is configured to be released from engagement with the plunger holder by displacement of the actuator sleeve towards the distally displaced position, thereby enabling proximal displacement of the plunger holder, the plunger rod and the rotator relative to the actuator, whereby the actuator is configured to engage with the guide structure of the rotator and to rotate the rotator relative to the plunger holder, releasing the plunger rod from the plunger holder. WO 2013/034984 A2 discloses another medicament delivery device with corresponding functionalities.

The configuration which provides the auto-penetration functionality may be rather bulky.

### SUMMARY

An object of the present disclosure is to provide a medicament delivery device which solves or at least mitigates problems of the prior art.

There is hence provided a medicament delivery device comprising: a housing, a delivery member cover slidably arranged in the housing, a medicament container housing arranged in the housing, a forward-biased rotator structure configured to move the medicament container housing in a forward direction in the housing, wherein the delivery member cover is configured to be moved axially from an extended position relative to the housing to a retracted position, thereby causing the rotator structure to rotate from a first rotator position to a second rotator position, and a forward-biased plunger rod extending axially inside rotator structure and having a first axial engagement position with the rotator structure when the rotator structure is in the first rotator position and the second rotator position, the rotator structure having a first rotator guide track structure configured to cooperate with an inner surface structure of the housing, enabling: rotation of the rotator structure when the rotator structure is rotated from the first rotator position to the second rotator position, preventing axial forward movement of the rotator structure inside the housing when the rotator structure is in the first rotator position, releasing of the rotator structure from axial engagement with the inner surface structure of the housing when the rotator structure is rotated from the first rotator position towards the second rotator position, causing the forward-biased rotator structure, the plunger rod and the medicament container housing to move a first forward distance in the housing to perform an auto-penetration operation.

The auto-penetration functionality is hence obtained by means of the rotator structure instead of relying on a triple-layer configuration of an actuator sleeve, an actuator holder and a plunger holder as in WO2017/198383. The three-layer arrangement of WO2017/198383 is hence replaced by fewer layers. The auto-penetration assembly may therefore be made more compact.

According to one embodiment the rotator structure is configured to cooperate with the inner surface structure of the housing to provide a final rotation of the rotator structure towards a third rotator position in an end stage of the auto-penetration operation, causing the plunger rod to be released from its engagement with the rotator structure in the first axial engagement position and move in the forward direction relative to the rotator structure.

According to one embodiment the rotator structure is configured to axially engage with the inner surface structure of the housing when the rotator structure has moved the first forward distance, thereby preventing further axial forward movement of the rotator structure and the medicament container housing in the housing.

According to one embodiment the rotator structure has an inner surface provided with a stepped structure and the plunger rod is provided with a radially outwards extending protrusion configured to cooperate with the stepped structure to enable axial engagement between the rotator structure and the plunger rod.

According to one embodiment the stepped structure has a first step on which the radially outwards extending protrusion rests when the rotator structure is in the first rotator position.

According to one embodiment the stepped structure includes an axially extending groove opening from the first step, the radially outwards extending protrusion being configured to move into the groove from the first step when the rotator structure is rotated from the second rotator position towards the third rotator position.

According to one embodiment the inner surface structure of the housing includes a radially inwards extending pin and the first rotator guide track structure comprises a circumferential radially outwards extending rib portion bearing against the pin when the rotator structure is rotated from the first rotator position towards the second rotator position, enabling the rotator structure to rotate relative to the housing but preventing it to move forward.

According to one embodiment the first rotator guide track structure comprises an axial radially outwards extending rib portion, the circumferential radially outwards extending rib portion transitioning to the axial radially outwards extending rib portion, enabling the forward-biased rotator structure to move forward relative to the housing when the rotator structure is moved from the first rotator position and the pin moves past the circumferential radially outwards extending rib portion.

According to one embodiment the first rotator guide track structure has a helical radially outwards extending rib portion, the axial radially outwards extending rib portion transitioning to the helical radially outwards extending rib portion, the helical radially outwards extending rib portion providing a final rotation of the rotator structure from the second rotator position towards the third rotator position.

According to one embodiment the helical radially outwards extending rib portion is provided on an outer surface of the rotator structure and has an axially extending component and a circumferentially extending component.

According to one embodiment the delivery member cover is configured to initially be arranged in an initial position in which it is arranged further in the housing than in the extended position, wherein the delivery member cover is configured to be moved from the initial position to the extended position, causing the rotator structure to rotate from an initial rotator position to the first rotator position.

According to one embodiment the plunger rod has an initial axial engagement position with the rotator structure when the rotator structure is in the initial rotator position, and wherein the plunger rod is configured to disengage from the initial axial engagement position and move forward axially a priming distance relative to the rotator structure and engage with the rotator structure in the first axial engagement position.

According to one embodiment the stepped structure has a second step on which the radially outwards extending protrusion rests when the rotator structure is in the initial rotator position.

According to one embodiment the first step is arranged closer to the front end of the housing than the second step.

According to one embodiment the rotator structure comprises a rotator and a rotator sleeve configured to be rotationally and axially locked to the rotator, the rotator sleeve being provided with the first rotator guide structure.

Generally, all terms used in the claims are to be interpreted according to their ordinary meaning in the technical field, unless explicitly defined otherwise herein. All references to "a/an/the element, apparatus, component, means, etc. are to be interpreted openly as referring to at least one instance of the element, apparatus, component, means, etc.", unless explicitly stated otherwise.

### BRIEF DESCRIPTION OF THE DRAWINGS

The specific embodiments of the inventive concept will now be described, by way of example, with reference to the accompanying drawings, in which:
Fig. 1 shows a perspective view of an example of a medicament delivery device in a pre-primed initial state;
Fig. 2 is an exploded view of the medicament delivery device in Fig. 1;
Fig. 13 is perspective view of the medicament delivery device in the initial state without the housing;

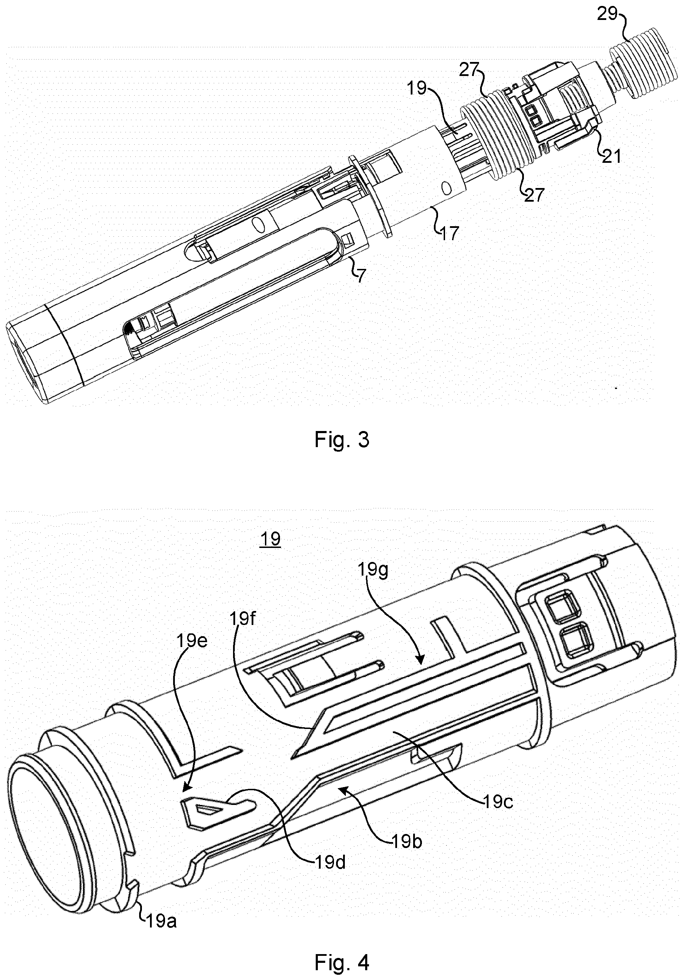

Fig. 4 shows an example of a rotator;

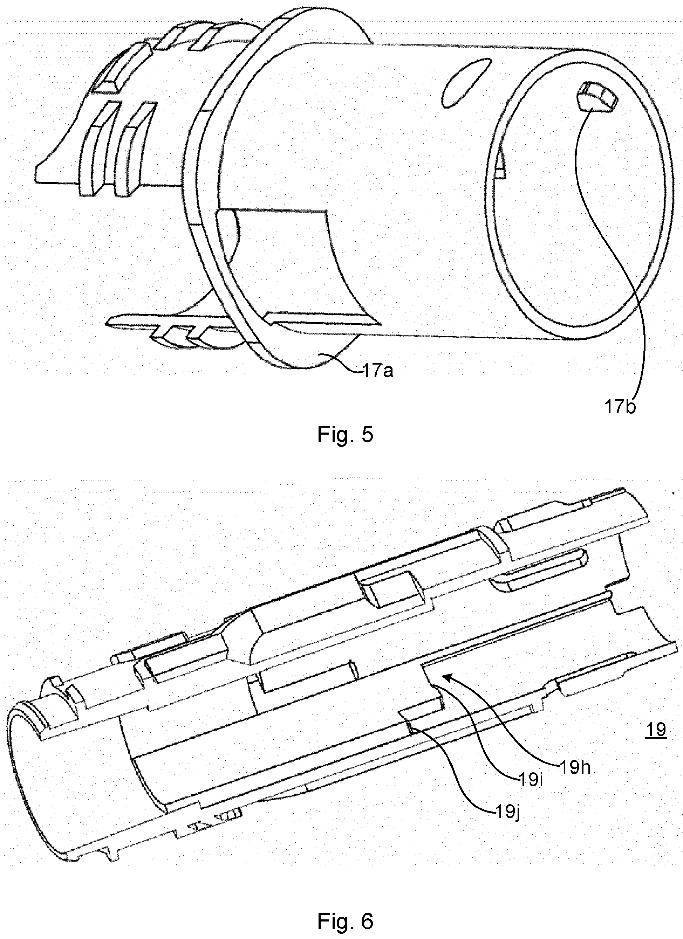

Fig. 5 is a perspective view of a sleeve;
Fig. 6 is a longitudinal section of the rotator;

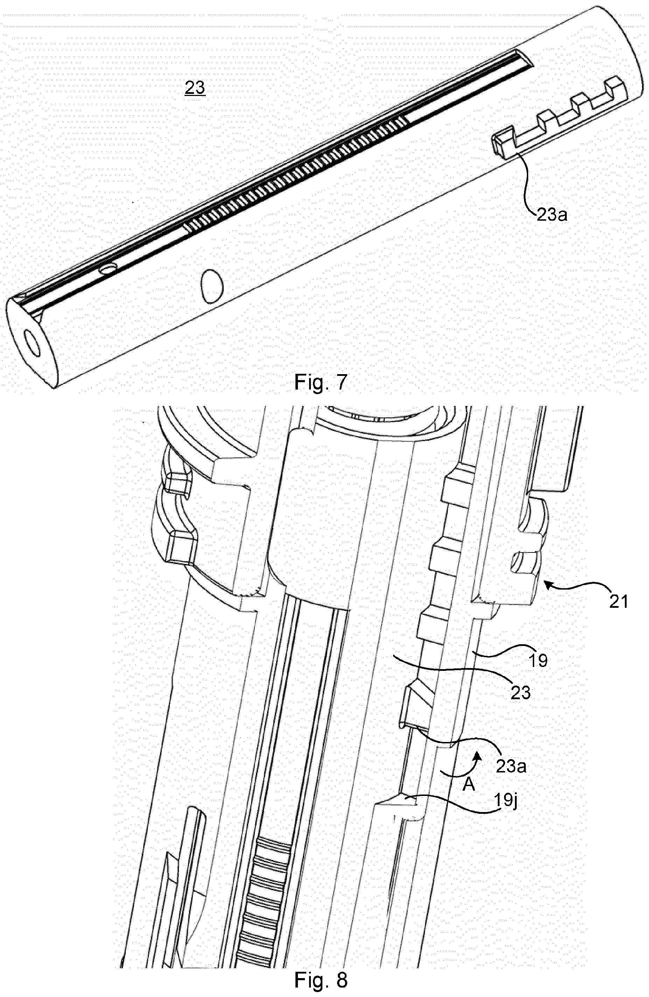

Fig. 7 is a perspective view of a plunger rod;
Fig. 8 is a longitudinal section showing interaction between the plunger rod and the rotator;

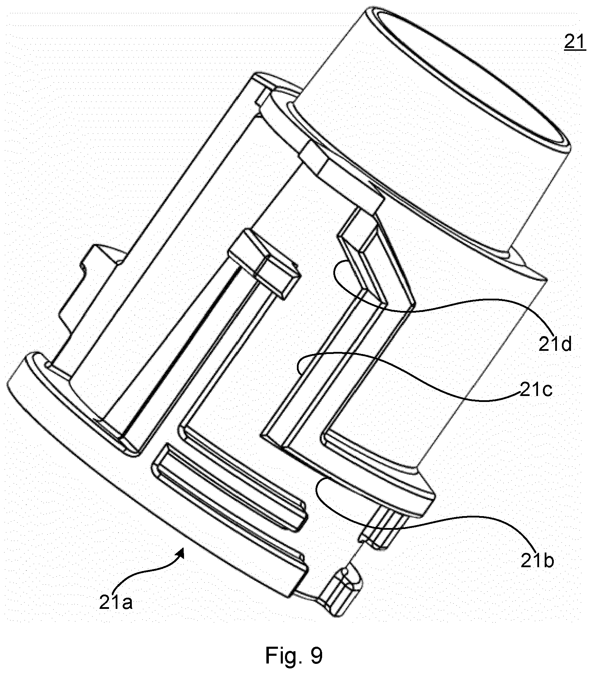

Fig. 9 is a perspective view of a rotator sleeve;

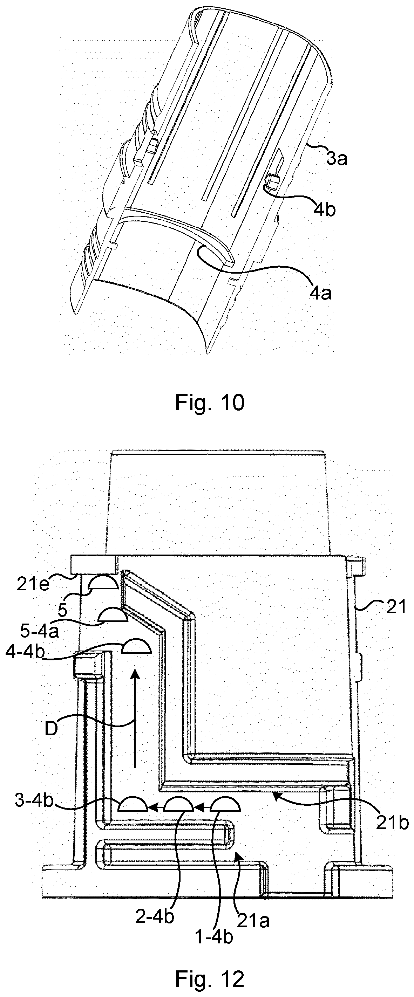

Fig. 10 is a longitudinal section of only the middle part of the housing;

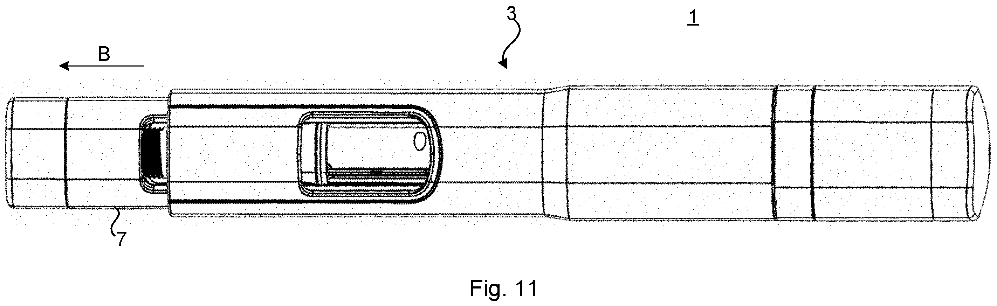

Fig. 11 shows the medicament delivery device in a primed state;
Fig. 12 is a side view of the rotator sleeve indicating its relative positions to the housing in different states of the medicament delivery device;

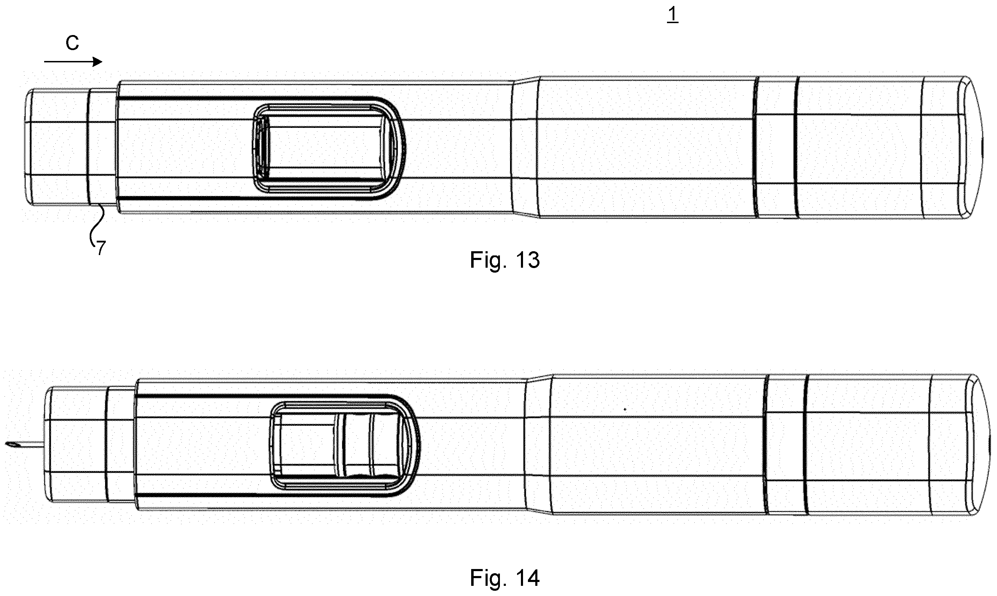

Fig. 13 shows the medicament delivery device when arranged against a site of injection;
Fig. 14 depicts the medicament delivery device after auto-penetration;

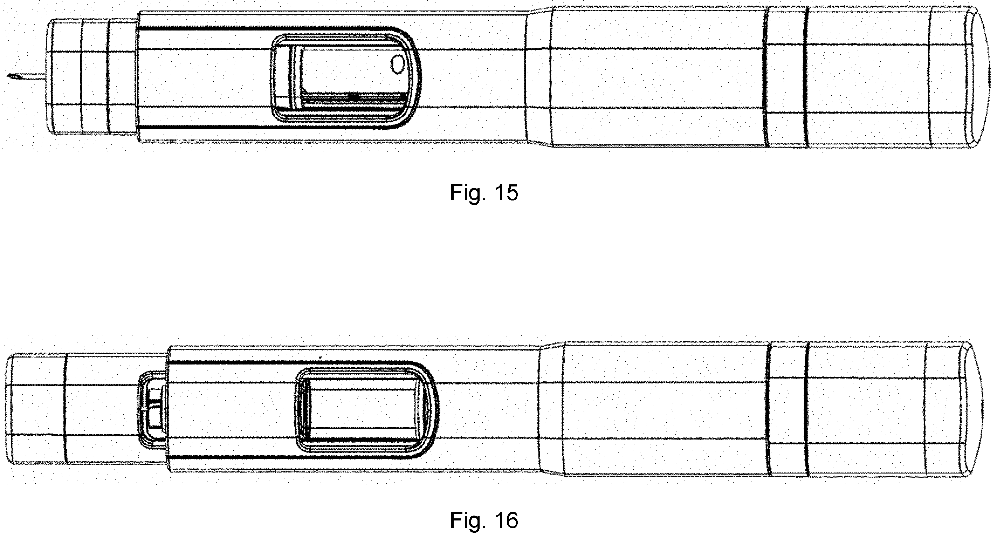

Fig. 15 shows the medicament delivery device during injection; and
Fig. 16 shows the medicament delivery device in a locked-out state after injection.

### DETAILED DESCRIPTION

The inventive concept will now be described more fully hereinafter with reference to the accompanying drawings, in which exemplifying embodiments are shown. The inventive concept may, however, be embodied in many different forms and should not be construed as limited to the embodiments set forth herein; rather, these embodiments are provided by way of example so that this disclosure will be thorough and complete, and will fully convey the scope of the inventive concept to those skilled in the art. Like numbers refer to like elements throughout the description.

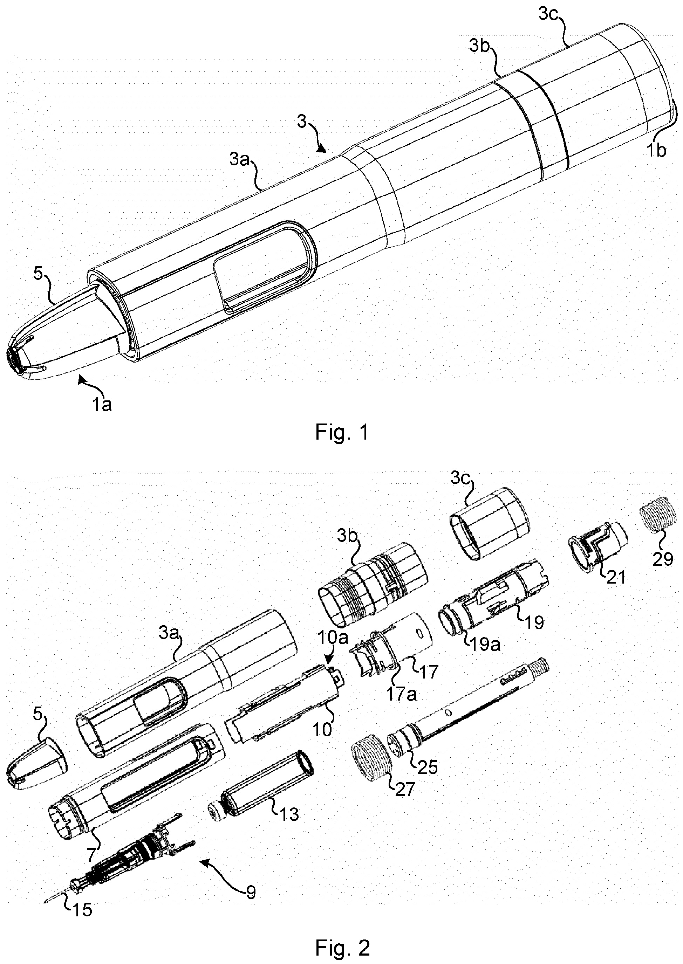

Fig. 1 depicts an example of a medicament delivery device 1. The medicament delivery device 1 may for example be an auto-injector.

The medicament delivery device 1 comprises a housing 3. In the present example, the housing 3 has a front part 3a, a rear part 3c and a middle part 3b arranged between the front part 3a and the rear part 3c.

The medicament delivery device 1 has a front end 1a and a rear end 1b. The medicament delivery device 1 has a detachable cap 5. The cap 5 forms part of the front end 1a of the medicament delivery device 1 when the cap 5 is attached to the housing 3. The front end 1a means that end of the medicament delivery device 1 which faces the site of injection when in use. The rear end 1b is the opposite end of the medicament delivery device 1 relative to the front end 1a. The front end 1a may alternatively be referred to as the proximal end of the medicament delivery device 1. The rear end 1b may alternatively be referred to as the distal end of the medicament delivery device 1.

Fig. 2 depicts an exploded view of the medicament delivery device 1. The medicament delivery device 1 comprises a delivery member cover 7 arranged in the housing 3. The delivery member cover 7 is slidably arranged in the housing 3. The delivery member 7 is configured to move axially relative to the housing 3 between an extended position and a retracted position. In the extended position the delivery member cover 7 protrudes more from the front end of the housing 3 than in the retracted position. The delivery member cover 7 is configured to be biased towards the extended position.

The medicament delivery member 1 may be provided with a priming functionality. The medicament delivery device 1 may hence initially be arranged in an initial state which is an un-primed state. The medicament delivery device 1 may be in this state before the cap 5 is removed. In this case, the cap 5 which is attached to the front part 3a of the housing 3 presses the delivery member cover 7 inwards into the housing 3 towards the rear end 1b of the medicament delivery device 1. The delivery member cover 7 is thus arranged in an initial position. When the cap 5 is removed, the delivery member cover 7 is released and due to forward-biasing moved axially from the initial position to the extended position.

The medicament delivery device 1 comprises a medicament container housing 10 configured to receive a medicament container 13, and a delivery member 15 such as a needle, for example a double-sided needle, configured to be in fluid connection with the medicament container.

The medicament delivery device 1 furthermore comprises a medicament delivery assembly 9. The delivery member 13 forms part of the medicament delivery assembly 9. In case the medicament delivery device 1 is provided with priming functionality, the delivery member 15 may be a double-sided needle that may be moved rearwards, towards the rear end 1b of the medicament delivery device 1, when the delivery member cover 7 is moved from the initial position towards the extended position thereby rupturing a septum arranged in or on the medicament container. Since this aspect does not form part of the present disclosure, and since many different variations of such priming functionality can be realised, this feature will not be discussed in any further detail herein.

The present example of the medicament delivery device 1 comprises a sleeve 17, a rotator 19 and a rotator sleeve 21. The rotator 19 and the rotator sleeve 21 form a rotator structure. The rotator 19 and the rotator sleeve 21 are rotationally locked relative to each other. Rotation of the rotator 19 hence causes rotation of the rotator sleeve 21 with the same amount of rotation as the rotator 19. The rotator 19 and the rotator sleeve 21 are axially locked relative to each other. Thus, axial movement of the rotator 19 causes the same amount of axial movement of the rotator sleeve 21 and vice versa. The rotator 19 and the rotator sleeve 21 engage with each other to provide axial and rotational locking between the rotator 19 and the rotator sleeve 21. The rotator sleeve 21 is arranged closer to the rear end 1b of the medicament delivery device 1 than the rotator 19. The rotator structure according to the present example hence comprises two components, namely the rotator 19 and the rotator sleeve 21. Alternatively, the rotator structure could for example comprise a single component. The rotator 19 and the rotator sleeve 21 could for example be integral, i.e. forming a single component. Furthermore, the sleeve 17 may according to one variation be discarded with. In this case, the delivery member cover 7 may be configured to directly cooperate with the rotator structure.

The medicament delivery device 1 comprises a plunger rod 23 extending through the rotator 19. The plunger rod 23 is configured to be forward-biased. The plunger rod 23 is hence configured to be biased towards the front end 1a of the medicament delivery device 1. The medicament delivery device 1 comprises a plunger 25 provided on a front end of the plunger rod 23. The plunger rod 23 and the plunger 25 are configured to extend into the medicament container 13.

The exemplified medicament delivery device 1 comprises a first resilient member 27 configured to bias the delivery member cover 7 towards the extended position. The first resilient member 27 may for example be a spring.

The medicament delivery device 1 comprises a second resilient member 29 configured to bias the rotator structure in the forward direction, i.e. towards the front end of the medicament delivery device 1. The second resilient member 29 is configured to bias the rotator sleeve 21 towards the front end of the medicament delivery device 1. The second resilient member 29 may for example be a spring. The rear end of the second resilient member 29 may be in contact with the rear end 1b of the medicament delivery device 1.

The sleeve 17 is axially locked relative to the delivery member cover 7. The delivery member cover 7 is configured to engage with the sleeve 17 to axially lock the delivery member cover 7 with the sleeve 17. Axial movement of the delivery member cover 7 hence causes axial movement of the sleeve 17. The sleeve 17 has a radially outwards protruding holding structure 17a in mechanical contact with the front end of the first resilient member 27, providing biasing of the sleeve 17 towards the front end 1a of the medicament delivery device 1. The middle part 3b of the housing 3 may have an inner structure in mechanical contact with the rear end of the first resilient member 27. The first resilient member 27 is hence compressed between the holding structure 17a and the inner structure.

The medicament container housing 10 is configured to be axially locked relative to the rotator structure. The medicament container housing 10 is configured to engage with the rotator structure. In the example in Fig. 2, the medicament container housing 10 is configured to engage with the rotator 19. Thus, axial movement of the rotor 19 causes corresponding axial movement of the medicament container housing 10. The rotator 19 is configured to rotate relative to the medicament container housing 10. The rotator 19 may be provided with a circumferentially extending rib 19a and the medicament container housing 10 may have corresponding arms 10a configured to engage with the rib 19a to prevent relative axial movement between the medicament container housing 10 and the rotator 19 but to enable relative rotation of the rotator 19.

Fig. 3 shows the medicament delivery device 1 in the initial state without the housing 3.

Fig. 4 is a perspective view of the rotator 19. The rotator 19 is hollow and has a generally cylindrical shape and extends along the longitudinal axis of the medicament delivery device 1. The rotator 19 has a main rotator guide track structure 19b provided on the external surface of the rotator 19. The main rotator guide track structure 19b is configured to engage with an inner engagement structure of the sleeve 17. The inner engagement structure is a radially inwards extending structure such as a pin. The main rotator guide track structure 19b is configured to transform linear motion of the sleeve 17 to rotational motion of the rotator 19. The main rotator guide track structure 19b has a first axial track 19c in which the engagement structure of the sleeve 17 is arranged in the initial state of the medicament delivery device 1. The engagement structure is in the initial state arranged in a rear portion of the first axial track 19c. The main rotator guide track structure 19b comprises a first helical portion 19d arranged in the forward direction relative to the first axial track 19c and facing the first axial track 19c. The first helical portion 19d leads to an opening 19e, enabling the engagement structure to move further forward relative to the first helical portion 19d. The main rotator guide track structure 19b comprises a second helical portion 19f facing the opening 19e. The second helical portion 19f is helical in an opposite direction compared to the first helical portion 19d. The second helical portion 19f transitions to a second axial track 19g extending parallel with the first axial track 19c and extending towards the rear end 1b of the medicament delivery device 1.

When the delivery member cover 7 is moved from the initial position to the extended position, the rotator 19 is rotated from an initial rotator position to a first rotator position.

Fig. 5 shows a perspective view of the sleeve 17. The aforementioned protruding holding structure 17a also functions as a stop. When the sleeve 17 is moved axially towards the front end 1a of the medicament delivery device, after a certain amount of movement the protruding holding structure 17a will hit inner ribs of the front part 3a of the housing 3. The sleeve 17 is thereby prevented from further movement in the forward direction.

The engagement structure 17b arranged on the inner surface of the sleeve 17 is configured to cooperate with the main rotator guide track structure 19b.

Fig. 6 shows a section of the rotator 19. The rotator 19 has an inner surface provided with a stepped structure 19h or stair-like configuration. The stepped structure includes two steps 19i and 19j.

Fig. 7 shows the plunger rod 23. The plunger rod 23 is provided with a radially outwards extending protrusion 23a. The protrusion 23a is configured to cooperate with the inner rotator structure 19h. In the initial state of the medicament delivery device 1, the protrusion 23a is configured to bear against or rest on the second step 19i. The plunger rod 23 hence has an initial axial engagement position with the rotator 19 when the protrusion 23a rests on the second step 19i. When the cap 5 is removed from the housing 3, the delivery member cover 7 is configured to move from the initial position towards the extended position. This forward movement of the delivery member cover 7 causes forward movement of the sleeve 17. The cooperation between the sleeve 17 and the rotator 19, as the engagement structure 17b moves from the first axial track 19c to the first helical portion 19d, causes rotation of the rotator 19. The main rotator guide track structure 19b is designed such that the amount of rotation of the rotator 19 will cause the protrusion 23a to move from the second step 19i to the first step 19j. When the protrusion 23a rests on the first step 19j, the plunger rod 23 has a first axial engagement position with the rotator 19. The plunger rod 23 will thereby move forward inside the medicament container 13 and perform a priming operation. Any air in the medicament container 13 will thereby be expelled from the medicament container 13.

As an alternative to the above configuration, the rotator may be have an inner surface provided with a radially inwards extending protrusion and the plunger rod may be provided with the stepped structure.

Fig. 8 shows the plunger rod 23 when the plunger rod protrusion 23a rests on the second step 19i. The direction of rotation of the rotator 19 from the initial rotator position towards the first rotator position when the delivery member cover 7 moves from the initial position towards the extended position is shown by arrow A.

Fig. 9 is a perspective view of the rotator sleeve 21. The rotator sleeve 21 is provided with a first rotator guide track structure 21a. The first rotator guide track structure 21a is arranged on the outer surface of the rotator sleeve 21.

The first rotator guide track structure 21a comprises a circumferentially radially outwards extending rib portion 21b. The first rotator guide track structure 21a comprises an axial radially outwards extending rib portion 21c. The circumferentially radially outwards extending rib portion 21b transitions to the axial radially outwards extending rib portion 21c. The axially radially outwards extending rib portion 21c extends rearwards relative to the circumferentially radially outwards extending rib portion 21c. The first rotator guide track structure 21a comprises an inclined or helical radially outwards extending rib portion 21d. The axially outwards extending rib portion 21c transitions to the helical radially outwards extending rib portion 21d. The helical radially outwards extending rib portion 21d is arranged rearwards relative to the axially outwards extending rib portion 21c.

Fig. 10 shows a longitudinal section of the middle part 3b of the housing 3. The inner structure 4a which is in mechanical contact with the rear end of the first resilient member 27 can here be seen. The inner structure 4 is a radially inwards extending structure such as a rib. The housing 3 has an inner surface structure 4b. In the example, the middle part 3b is provided with the inner surface structure 4b. The inner surface structure 4b extends radially inwards. The inner surface structure 4b may for example be a protrusion such as a pin, as required by the invention.

The inner surface structure 4b is configured to cooperate with the first rotator guide track structure 21a to control the rotational and axial movement of the rotator sleeve 21 and hence of the rotator 19 and the medicament container housing 10 which is axially locked to the rotator 19. This enables priming and auto-penetration functionality of the medicament delivery device 1.

Fig. 11 depicts the medicament delivery device 1 in a primed state. The primed state is obtained by removing the cap 5 from the housing 3. By removing the cap 5, the forward-biased delivery member cover 7 is able to move forward to the extended position as shown by arrow B. This causes the sleeve 17 to move forward, thereby rotating the rotator 19. As the rotator 19 is rotated from the initial rotator position towards the first rotator position, the rotator 19 is rotated relative to the plunger rod 23. The plunger rod 23 will thereby be moved forward as the protrusion 23 is moved from the second step 19i a priming distance corresponding to the axial distance between the steps 19i and 19j to the first step 19j.

Fig. 12 shows the rotator sleeve 21 and the various positions that the inner surface structure 4b obtains as the medicament delivery device 1 attains its different states as will be described in the following.

In the initial state, prior to priming, the inner surface structure 4b is in a first position 1-4b relative to the first rotator guide track structure 21a. In the first position 1-4b the inner surface structure 4b rests against the circumferentially radially outwards extending rib portion 21b. The circumferentially radially outwards extending rib portion 21b prevents axial movement of the rotator sleeve 21 as long as the inner surface structure 4b bears against a front surface of the circumferentially radially outwards extending rib portion 21b.

Fig. 12 also shows the second position 2-4b of the inner surface structure 4b, which is obtained when the medicament delivery device 1 has been primed and the delivery member cover 7 has attained its extended position. The rotator sleeve 21 has been rotated by the rotation of the rotator 19 from the initial position to the first rotator position. This rotation is enabled by the circumferentially radially outwards extending rib portion 21b, which allows the rotator sleeve 21 to rotate concurrently with the rotator 19. The inner surface structure 4b has thus obtained the second position 2-4b relative to the rotator sleeve 21. The inner surface structure 4b still bears against the circumferentially radially outwards extending rib portion 21b, preventing axial movement of the rotator sleeve 21.

Fig. 13 shows the medicament delivery device 1 when the delivery member cover 7 has been moved from the extended position to the retracted position, as indicated by arrow C. This situation typically occurs when a user presses the delivery member cover 7 towards the site of injection to inject the medicament contained in the medicament container. The rearward movement of the delivery member cover 7 causes the sleeve 17 to move towards the rear end 1b of the medicament delivery device 1 and the engagement structure 17a to move towards the second helical portion 19f. This causes rotation of the rotator 19 from the first rotator position to the second rotator position. The engagement structure 17a will then follow the second axial track 19g.

Fig. 12 shows the third position 3-4b of the inner surface structure 4b of the housing 3 when the delivery member cover 7 has obtained its retracted position. In the third position 3-4b, the inner surface structure 4b disengages from the rotator sleeve 21 as it moves past the circumferentially radially outwards extending rib portion 21b in the circumferential direction and to the axially outwards extending rib portion 21c. The rotator sleeve 21 is thereby able to move axially forward inside the housing 3 as indicated by the arrow D. The rotator 19 which is axially locked with the rotator sleeve 21 and the medicament container housing 10 are moved forward together with the rotator sleeve 21.

The rotation of the rotator 19 caused by the retraction of the delivery member cover 7 will not release the plunger rod 23 from engagement with the rotator 19. This rotation will move the protrusion 23a along the first step 19j but the protrusion 23a will not clear the first step 19j. Adjacent to the first step 19j is an axially extending groove or channel configured to receive the protrusion 23a therein. The first step 19j hence opens into the axially extending groove. In an end stage of the auto-penetration operation, shown in Fig. 14, when the rotator sleeve 21 has moved a first forward distance in the housing to perform an auto-penetration operation, the inner surface structure 4b reaches the inclined radially outwards extending rib portion 21d as illustrated by positions 4-4b and 5-4b in Fig. 12, which provides a final rotation of the rotator sleeve 21. The final rotation also provides a final rotation of the rotator 19 which thereby obtains a third rotator position, causing the protrusion 23a to clear the first step 19j and to move into the axially extending groove. The plunger rod 23 is hence released from the first axial engagement position with the rotator 19. This causes the plunger rod 23 to move forward in the housing 3 relative to the rotator 19, as shown in Fig. 1₅. About simultaneously with this, the rotator sleeve 21 has moved a first forward distance, wherein the inner surface structure 4b reaches a stop 21e provided on the rotator sleeve 21 causing forward movement of the rotator sleeve 21 and hence of the rotator 19 and the medicament container housing 10 to stop.

As previously mentioned, the protruding holding structure 17a also functions as a stop, which after the delivery member cover 7 has been released from its retracted position when medicament administration has been performed and the sleeve 17 has been moved a certain distance forward together with the delivery member cover 7, will hit inner ribs of the front part 3a of the housing 3, preventing further forward movement of the delivery member cover 7 and the sleeve 17, as shown in Fig. 16.

The inventive concept has mainly been described above with reference to a few examples. However, as is readily appreciated by a person skilled in the art, other embodiments than the ones disclosed above are equally possible within the scope of the inventive concept, as defined by the appended claims.

## Claims

1. A medicament delivery device (1) comprising:
- a housing (3),
- a delivery member cover (7) slidably arranged in the housing (3),
- a medicament container housing (10) arranged in the housing (3),
- a forward-biased rotator structure (19, 21) configured to move the medicament container housing (10) in a forward direction in the housing (3),
- a resilient member (29) configured to bias the rotator structure in the forward direction,
wherein the delivery member cover (7) is configured to be moved axially from an extended position relative to the housing (3) to a retracted position, thereby causing the rotator structure (19, 21) to rotate from a first rotator position to a second rotator position, and
- a forward-biased plunger rod (23) extending axially inside the rotator structure (19, 21) and having a first axial engagement position with the rotator structure (19, 21) when the rotator structure (19, 21) is in the first rotator position and the second rotator position,
the rotator structure (19, 21) having a first rotator guide track structure (21a) configured to cooperate with an inner surface structure (4b) of the housing (3), enabling:
rotation of the rotator structure (19, 21) when the rotator structure (19, 21) is rotated from the first rotator position to the second rotator position,
preventing axial forward movement of the rotator structure (19, 21) inside the housing (3) when the rotator structure (19, 21) is in the first rotator position,
releasing of the rotator structure (19, 21) from axial engagement with the inner surface structure (4b) of the housing (3) when the rotator structure (19, 21) is rotated from the first rotator position towards the second rotator position, causing the forward-biased rotator structure (19, 21), the plunger rod (23) and the medicament container housing (10) to move a first forward distance in the housing (3) to perform an auto-penetration operation
wherein the inner surface structure (4b) of the housing (3) includes a radially inwards extending pin and the first rotator guide track structure (21a) comprises a circumferential radially outwards extending rib portion (21b) bearing against the pin when the rotator structure (19, 21) is rotated from the first rotator position towards the second rotator position, enabling the rotator structure (19, 21) to rotate relative to the housing (3) but preventing it to move forward,
wherein the first rotator guide track structure (21a) comprises an axial radially outwards extending rib portion (21c), the circumferential radially outwards extending rib portion (21b) transitioning to the axial radially outwards extending rib portion (21c), enabling the forward-biased rotator structure (19, 21) to move forward relative to the housing (3) when the rotator structure (19, 21) is moved from the first rotator position and the pin moves past the circumferential radially outwards extending rib portion (21b).

2. The medicament delivery device (1) as claimed in claim 1, wherein the rotator structure (19, 21) is configured to cooperate with the inner surface structure (4b) of the housing (3) to provide a final rotation of the rotator structure (19, 21) towards a third rotator position in an end stage of the auto-penetration operation, causing the plunger rod (23) to be released from its engagement with the rotator structure (19, 21) in the first axial engagement position and move in the forward direction relative to the rotator structure (19, 21).

3. The medicament delivery device (1) as claimed in any of the preceding claims, wherein the rotator structure (19, 21) is configured to axially engage with the inner surface structure (4b) of the housing (3) when the rotator structure (19, 21) has moved the first forward distance, thereby preventing further axial forward movement of the rotator structure (19, 21) and the medicament container housing (10) in the housing (3).

4. The medicament delivery device (1) as claimed in any of the preceding claims, wherein the rotator structure (19, 21) has an inner surface provided with a stepped structure and the plunger rod (23) is provided with a radially outwards extending protrusion (23a) configured to cooperate with the stepped structure to enable axial engagement between the rotator structure (19, 21) and the plunger rod (23).

5. The medicament delivery device (1) as claimed in claim 4, wherein the stepped structure has a first step (19j) on which the radially outwards extending protrusion (23a) rests when the rotator structure (19, 21) is in the first rotator position.

6. The medicament delivery device (1) as claimed in claim 4 or 5, dependent of claim 2, wherein the stepped structure includes an axially extending groove opening from the first step (19j), the radially outwards extending protrusion (23a) being configured to move into the groove from the first step (19j) when the rotator structure (19, 21) is rotated from the second rotator position towards the third rotator position.

7. The medicament delivery device (1) as claimed in any of the preceding claims, wherein the first rotator guide track structure (21a) has a helical radially outwards extending rib portion (21d), the axial radially outwards extending rib portion (21c) transitioning to the helical radially outwards extending rib portion (21d), the helical radially outwards extending rib portion (21d) providing a final rotation of the rotator structure (19, 21) from the second rotator position towards the third rotator position.

8. The medicament delivery device (1) as claimed in claim 7, wherein the helical radially outwards extending rib portion (21d) is provided on an outer surface of the rotator structure (19, 21) and has an axially extending component and a circumferentially extending component.

9. The medicament delivery device (1) as claimed in any of the preceding claims, wherein the delivery member cover (7) is configured to initially be arranged in an initial position in which it is arranged further in the housing (3) than in the extended position, wherein the delivery member cover (7) is configured to be moved from the initial position to the extended position, causing the rotator structure (19, 21) to rotate from an initial rotator position to the first rotator position.

10. The medicament delivery device (1) as claimed in claim 9, wherein the plunger rod (23) has an initial axial engagement position with the rotator structure (19, 21) when the rotator structure (19, 21) is in the initial rotator position, and wherein the plunger rod (23) is configured to disengage from the initial axial engagement position and move forward axially a priming distance relative to the rotator structure (19, 21) and engage with the rotator structure (19, 21) in the first axial engagement position.

11. The medicament delivery device (1) as claimed in claim 10 dependent of claim 4, wherein the stepped structure has a second step (19i) on which the radially outwards extending protrusion (23a) rests when the rotator structure (19, 21) is in the initial rotator position.

12. The medicament delivery device (1) as claimed in claim 11, wherein the first step (19j) is arranged closer to the front end (1a) of the housing (3) than the second step (19i).

13. The medicament delivery device (1) as claimed in any of the preceding claims, wherein the rotator structure (19, 21) comprises a rotator (19) and a rotator sleeve (21) configured to be rotationally and axially locked to the rotator (19), the rotator sleeve (21) being provided with the first rotator guide structure (21).

## Patentansprüche

1. Arzneimittelabgabevorrichtung (1), umfassend:
- ein Gehäuse (3),
- eine in dem Gehäuse (3) verschiebbar angeordnete Abgabeelementabdeckung (7),
- ein in dem Gehäuse (3) angeordnetes Arzneimittelbehältergehäuse (10),
- eine vorwärts vorgespannte Rotatorstruktur (19, 21), die konfiguriert ist, um das Arzneimittelbehältergehäuse (10) in dem Gehäuse (3) in eine Vorwärtsrichtung zu bewegen,
- ein elastisches Element (29), das konfiguriert ist, um die Rotatorstruktur in die Vorwärtsrichtung vorzuspannen,
wobei die Abgabeelementabdeckung (7) konfiguriert ist, um axial von einer ausgefahrenen Position relativ zu dem Gehäuse (3) in eine eingefahrene Position bewegt zu werden, wobei dadurch die Rotatorstruktur (19, 21) veranlasst wird, von einer ersten Rotatorposition in eine zweite Rotatorposition zu rotieren, und
- eine vorwärts vorgespannte Kolbenstange (23), die axial innerhalb der Rotatorstruktur (19, 21) verläuft und eine erste axiale Eingriffsposition mit der Rotatorstruktur (19, 21) aufweist, wenn die Rotatorstruktur (19, 21) in der ersten Rotatorposition und der zweiten Rotatorposition ist,
wobei die Rotatorstruktur (19, 21) eine erste Rotatorführungsbahnstruktur (21a) aufweist, die konfiguriert ist, um mit einer Struktur (4b) einer inneren Oberfläche des Gehäuses (3) zusammenzuwirken, wodurch ermöglicht wird:
Rotation der Rotatorstruktur (19, 21), wenn die Rotatorstruktur (19, 21) von der ersten Rotatorposition in die zweite Rotatorposition rotiert wird,
Verhindern einer axialen Vorwärtsbewegung der Rotatorstruktur (19, 21) innerhalb des Gehäuses (3), wenn die Rotatorstruktur (19, 21) in der ersten Rotatorposition ist,
Lösen der Rotatorstruktur (19, 21) aus dem axialen Eingriff mit der Struktur (4b) der inneren Oberfläche des Gehäuses (3), wenn die Rotatorstruktur (19, 21) von der ersten Rotatorposition zu der zweiten Rotatorposition hin rotiert wird, was die nach vorwärts vorgespannte Rotatorstruktur (19, 21), die Kolbenstange (23) und das Arzneimittelbehältergehäuse (10) veranlasst, sich um eine erste Vorwärtsstrecke in dem Gehäuse (3) zu bewegen, um einen Autopenetrationsvorgang durchzuführen,
wobei die Struktur (4b) der inneren Oberfläche des Gehäuses (3) einen radial nach innen verlaufenden Stift einschließt und die erste Rotatorführungsbahnstruktur (21a) einen umlaufenden, radial nach außen verlaufenden Rippenabschnitt (21b) umfasst, der gegen den Stift drückt, wenn die Rotatorstruktur (19, 21) von der ersten Rotatorposition zu der zweiten Rotatorposition hin rotiert wird, wodurch ein Rotieren der Rotatorstruktur (19, 21) relativ zu dem Gehäuse (3) ermöglicht wird, ein Vorwärtsbewegen jedoch verhindert wird,
wobei die erste Rotatorführungsbahnstruktur (21a) einen axialen, radial nach außen verlaufenden Rippenabschnitt (21c) umfasst, wobei der in Umfangsrichtung radial nach außen verlaufende Rippenabschnitt (21b) in den axialen, radial nach außen verlaufenden Rippenabschnitt (21c) übergeht, wodurch die nach vorne gerichtete Rotatorstruktur (19, 21) sich relativ zu dem Gehäuse (3) vorwärts bewegen kann, wenn die Rotatorstruktur (19, 21) aus der ersten Rotatorposition bewegt wird und der Stift sich an dem in Umfangsrichtung radial nach außen verlaufenden Rippenabschnitt (21b) vorbei bewegt.

2. Arzneimittelabgabevorrichtung (1) nach Anspruch 1, wobei die Rotatorstruktur (19, 21) konfiguriert ist, um mit der Struktur (4b) der inneren Oberfläche des Gehäuses (3) zusammenzuwirken, um in einer Endphase des Autopenetrationsvorgangs eine abschließende Rotation der Rotatorstruktur (19, 21) zu einer dritten Rotatorposition hin bereitzustellen, wodurch die Kolbenstange (23) aus ihrem Eingriff mit der Rotatorstruktur (19, 21) in der ersten axialen Eingriffsposition gelöst wird und sich in die Vorwärtsrichtung relativ zu der Rotatorstruktur (19, 21) bewegt.

3. Arzneimittelabgabevorrichtung (1) nach einem der vorstehenden Ansprüche, wobei die Rotatorstruktur (19, 21) konfiguriert ist, um axial mit der Struktur (4b) der inneren Oberfläche des Gehäuses (3) in Eingriff zu stehen, wenn sich die Rotatorstruktur (19, 21) die erste Vorwärtsstrecke bewegt hat, wobei dadurch eine weitere axiale Vorwärtsbewegung der Rotatorstruktur (19, 21) und des Arzneimittelbehältergehäuses (10) in dem Gehäuse (3) verhindert wird.

4. Arzneimittelabgabevorrichtung (1) nach einem der vorstehenden Ansprüche, wobei die Rotatorstruktur (19, 21) eine innere Oberfläche aufweist, die mit einer gestufte Struktur versehen ist und die Kolbenstange (23) mit einem radial nach außen verlaufenden Vorsprung (23a) versehen ist, der konfiguriert ist, um mit der gestufte Struktur zusammenzuwirken, um einen axialen Eingriff zwischen der Rotatorstruktur (19, 21) und der Kolbenstange (23) zu ermöglichen.

5. Arzneimittelabgabevorrichtung (1) nach Anspruch 4, wobei die gestufte Struktur eine erste Stufe (19j) aufweist, auf der der radial nach außen verlaufende Vorsprung (23a) aufliegt, wenn sich die Rotatorstruktur (19, 21) in der ersten Rotatorposition befindet.

6. Arzneimittelabgabevorrichtung (1) nach Anspruch 4 oder 5, abhängig von Anspruch 2, wobei die gestufte Struktur eine sich axial verlaufende Nut einschließt, die sich von der ersten Stufe (19j) aus öffnet, wobei der sich radial nach außen verlaufende Vorsprung (23a) konfiguriert ist, um sich in die Nut von der ersten Stufe (19j) hinein zu bewegen, wenn die Rotatorstruktur (19, 21) von der zweiten Rotatorposition zu der dritten Rotatorposition hin rotiert wird.

7. Arzneimittelabgabevorrichtung (1) nach einem der vorstehenden Ansprüche, wobei die erste Rotatorführungsbahnstruktur (21a) einen spiralförmigen, radial nach außen verlaufenden Rippenabschnitt (21d) aufweist, wobei der axiale, radial nach außen verlaufende Rippenabschnitt (21c) in den spiralförmigen, radial nach außen verlaufenden Rippenabschnitt (21d) übergeht, wobei der spiralförmige, radial nach außen verlaufende Rippenabschnitt (21d) eine abschließende Rotation der Rotatorstruktur (19, 21) von der zweiten Rotatorposition zu der dritten Rotatorposition hin ermöglicht.

8. Arzneimittelabgabevorrichtung (1) nach Anspruch 7, wobei der spiralförmige, radial nach außen verlaufende Rippenabschnitt (21d) an einer äußeren Oberfläche der Rotatorstruktur (19, 21) bereitgestellt ist und eine axial verlaufende Komponente und eine in Umfangsrichtung verlaufende Komponente aufweist.

9. Arzneimittelabgabevorrichtung (1) nach einem der vorstehenden Ansprüche, wobei die Abgabeelementabdeckung (7) konfiguriert ist, um zunächst in einer Ausgangsposition angeordnet zu sein, in der sie weiter in dem Gehäuse (3) als in der ausgefahrenen Position angeordnet ist, wobei die Abgabeelementabdeckung (7) konfiguriert ist, um von der Ausgangsposition in die ausgefahrene Position bewegt zu werden, wodurch die Rotatorstruktur (19, 21) von einer anfänglichen Rotatorposition in die erste Rotatorposition rotiert.

10. Arzneimittelabgabevorrichtung (1) nach Anspruch 9, wobei die Kolbenstange (23) eine anfängliche axiale Eingriffsposition mit der Rotatorstruktur (19, 21) aufweist, wenn sich die Rotatorstruktur (19, 21) in der anfänglichen Rotatorposition befindet, und wobei die Kolbenstange (23) konfiguriert ist, um sich aus der anfänglichen axialen Eingriffsposition zu lösen und sich axial um eine Voreilstrecke relativ zu der Rotatorstruktur (19, 21) vorwärts zu bewegen und mit der Rotatorstruktur (19, 21) in der ersten axialen Eingriffsposition in Eingriff zu kommen.

11. Arzneimittelabgabevorrichtung (1) nach Anspruch 10, abhängig von Anspruch 4, wobei die gestufte Struktur eine zweite Stufe (19i) aufweist, auf der der radial nach außen verlaufende Vorsprung (23a) ruht, wenn die Rotatorstruktur (19, 21) in der anfänglichen Rotatorposition ist.

12. Arzneimittelabgabevorrichtung (1) nach Anspruch 11, wobei die erste Stufe (19j) näher an dem vorderen Ende (1a) des Gehäuses (3) als die zweite Stufe (19i) angeordnet ist.

13. Arzneimittelabgabevorrichtung (1) nach einem der vorstehenden Ansprüche, wobei die Rotatorstruktur (19, 21) einen Rotator (19) und eine Rotatorhülse (21) umfasst, die konfiguriert ist, um mit dem Rotator (19) rotatorisch und axial verriegelt zu sein, wobei die Rotatorhülse (21) mit der ersten Rotatorführungsstruktur (21) versehen ist.

## Revendications

1. Dispositif de délivrance de médicament (1) comprenant :
- un logement (3),
- un couvercle d'élément de délivrance (7) agencé de manière coulissante dans le logement (3),
- un logement de récipient de médicament (10) agencé dans le logement (3),
- une structure de rotateur (19, 21) sollicitée vers l'avant, conçue pour déplacer le logement de récipient de médicament (10) dans une direction vers l'avant dans le logement (3),
- un élément élastique (29) conçu pour solliciter la structure de rotateur dans la direction vers l'avant,
dans lequel le couvercle d'élément de délivrance (7) est conçu pour être déplacé axialement d'une position étendue par rapport au logement (3) à une position rétractée, amenant de ce fait la structure de rotateur (19, 21) à effectuer une rotation d'une première position de rotateur à une deuxième position de rotateur, et
- une tige de piston (23) sollicitée vers l'avant s'étendant axialement à l'intérieur de la structure de rotateur (19, 21) et ayant une première position de mise en prise axiale avec la structure de rotateur (19, 21) lorsque la structure de rotateur (19, 21) est dans la première position de rotateur et la deuxième position de rotateur,
la structure de rotateur (19, 21) ayant une première structure de piste de guidage de rotateur (21a) conçue pour coopérer avec une structure de surface interne (4b) du logement (3), permettant :
une rotation de la structure de rotateur (19, 21) lorsque la structure de rotateur (19, 21) est mise en rotation de la première position de rotateur à la deuxième position de rotateur,
empêchant un déplacement axial vers l'avant de la structure de rotateur (19, 21) à l'intérieur du logement (3) lorsque la structure de rotateur (19, 21) est dans la première position de rotateur,
la libération de la structure de rotateur (19, 21) d'une mise en prise axiale avec la structure de surface interne (4b) du logement (3) lorsque la structure de rotateur (19, 21) est mise en rotation de la première position de rotateur vers la deuxième position de rotateur, amenant la structure de rotateur (19, 21) sollicitée vers l'avant, la tige de piston (23) et le logement de récipient de médicament (10) à se déplacer d'une première distance vers l'avant dans le logement (3) pour mettre en œuvre une opération d'auto-pénétration
dans lequel la structure de surface interne (4b) du logement (3) comporte une broche s'étendant radialement vers l'intérieur et la première structure de piste de guidage de rotateur (21a) comprend une partie de nervure circonférentielle s'étendant radialement vers l'extérieur (21b) s'appuyant contre la broche lorsque la structure de rotateur (19, 21) est mise en rotation de la première position de rotateur vers la deuxième position de rotateur, permettant à la structure de rotateur (19, 21) d'effectuer une rotation par rapport au logement (3) mais l'empêchant de se déplacer vers l'avant,
dans lequel la première structure de piste de guidage de rotateur (21a) comprend une partie de nervure axiale s'étendant radialement vers l'extérieur (21c), la partie de nervure circonférentielle s'étendant radialement vers l'extérieur (21b) effectuant une transition vers la partie de nervure axiale s'étendant radialement vers l'extérieur (21c), permettant à la structure de rotateur (19, 21) sollicitée vers l'avant de se déplacer vers l'avant par rapport au logement (3) lorsque la structure de rotateur (19, 21) est déplacée de la première position de rotateur et que la broche passe devant la partie de nervure circonférentielle s'étendant radialement vers l'extérieur (21b).

2. Dispositif de délivrance de médicament (1) selon la revendication 1, dans lequel la structure de rotateur (19, 21) est conçue pour coopérer avec la structure de surface interne (4b) du logement (3) pour fournir une rotation finale de la structure de rotateur (19, 21) en direction d'une troisième position de rotateur dans une phase finale de l'opération d'auto-pénétration, amenant la tige de piston (23) à être libérée de sa mise en prise avec la structure de rotateur (19, 21) dans la première position de mise en prise axiale et à se déplacer dans la direction vers l'avant par rapport à la structure de rotateur (19, 21).

3. Dispositif de délivrance de médicament (1) selon l'une quelconque des revendications précédentes, dans lequel la structure de rotateur (19, 21) est conçue pour venir en prise axialement avec la structure de surface interne (4b) du logement (3) lorsque la structure de rotateur (19, 21) s'est déplacée de la première distance vers l'avant, empêchant de ce fait un déplacement axial vers l'avant supplémentaire de la structure de rotateur (19, 21) et du logement de récipient de médicament (10) dans le logement (3).

4. Dispositif de délivrance de médicament (1) selon l'une quelconque des revendications précédentes, dans lequel la structure de rotateur (19, 21) a une surface interne pourvue d'une structure étagée et la tige de piston (23) est pourvue d'une partie saillante s'étendant radialement vers l'extérieur (23a) conçue pour coopérer avec la structure étagée pour permettre une mise en prise axiale entre la structure de rotateur (19, 21) et la tige de piston (23).

5. Dispositif de délivrance de médicament (1) selon la revendication 4, dans lequel la structure étagée a un premier étagement (19j) sur lequel la partie saillante s'étendant radialement vers l'extérieur (23a) repose lorsque la structure de rotateur (19, 21) est dans la première position de rotateur.

6. Dispositif de délivrance de médicament (1) selon la revendication 4 ou 5, en dépendance de la revendication 2, dans lequel la structure étagée comporte une ouverture de rainure s'étendant axialement à partir du premier étagement (19j), la partie saillante s'étendant radialement vers l'extérieur (23a) étant conçue pour se déplacer dans la rainure à partir du premier étagement (19j) lorsque la structure de rotateur (19, 21) est mise en rotation de la deuxième position de rotateur vers la troisième position de rotateur.

7. Dispositif de délivrance de médicament (1) selon l'une quelconque des revendications précédentes, dans lequel la première structure de piste de guidage de rotateur (21a) a une partie de nervure hélicoïdale s'étendant radialement vers l'extérieur (21d), la partie de nervure axiale s'étendant radialement vers l'extérieur (21c) effectuant une transition vers la partie de nervure hélicoïdale s'étendant radialement vers l'extérieur (21d), la partie de nervure hélicoïdale s'étendant radialement vers l'extérieur (21d) fournissant une rotation finale de la structure de rotateur (19, 21) de la deuxième position de rotateur vers la troisième position de rotateur.

8. Dispositif de délivrance de médicament (1) selon la revendication 7, dans lequel la partie de nervure hélicoïdale s'étendant radialement vers l'extérieur (21d) est fournie sur une surface externe de la structure de rotateur (19, 21) et a un composant s'étendant axialement et un composant s'étendant circonférentiellement.

9. Dispositif de délivrance de médicament (1) selon l'une quelconque des revendications précédentes, dans lequel le couvercle d'élément de délivrance (7) est conçu pour être initialement agencé dans une position initiale dans laquelle il est agencé plus loin dans le logement (3) que dans la position étendue, dans lequel le couvercle d'élément de délivrance (7) est conçu pour être déplacé de la position initiale à la position étendue, amenant la structure de rotateur (19, 21) à effectuer une rotation d'une position initiale de rotateur à la première position de rotateur.

10. Dispositif de délivrance de médicament (1) selon la revendication 9, dans lequel la tige de piston (23) a une position initiale de mise en prise axiale avec la structure de rotateur (19, 21) lorsque la structure de rotateur (19, 21) est dans la position initiale de rotateur, et dans lequel la tige de piston (23) est conçue pour se désaccoupler de la position initiale de mise en prise axiale et se déplacer vers l'avant axialement sur une distance d'amorçage par rapport à la structure de rotateur (19, 21) et venir en prise avec la structure de rotateur (19, 21) dans la première position de mise en prise axiale.

11. Dispositif de délivrance de médicament (1) selon la revendication 10 en dépendance de la revendication 4, dans lequel la structure étagée a un second étagement (19i) sur lequel la partie saillante s'étendant radialement vers l'extérieur (23a) repose lorsque la structure de rotateur (19, 21) est dans la position initiale de rotateur.

12. Dispositif de délivrance de médicament (1) selon la revendication 11, dans lequel le premier étagement (19j) est agencé plus près de l'extrémité avant (1a) du logement (3) que le second étagement (19i).

13. Dispositif de délivrance de médicament (1) selon l'une quelconque des revendications précédentes, dans lequel la structure de rotateur (19, 21) comprend un rotateur (19) et un manchon de rotateur (21) conçu pour être verrouillé en rotation et axialement au rotateur (19), le manchon de rotateur (21) étant pourvu de la première structure de guidage de rotateur (21).
